(19) **Europäisches Patentamt**

**European Patent Office**

**Office européen des brevets**

(11) **EP 1 458 698 B1**

(12) **EUROPEAN PATENT SPECIFICATION**

(45) Date of publication and mention
of the grant of the patent:
**20.04.2005 Bulletin 2005/16**

(51) Int Cl.$^7$: **C07D 301/10**

(86) International application number:
**PCT/US2002/037022**

(21) Application number: **02803665.5**

(22) Date of filing: **19.11.2002**

(87) International publication number:
**WO 2003/044002 (30.05.2003 Gazette 2003/22)**

(54) **A PROCESS AND SYSTEMS FOR THE EPOXIDATION OF AN OLEFIN**

VERFAHREN UND SYSTEME ZUR EPOXIDIERUNG EINES OLEFINS

PROCEDE ET SYSTEMES D'EPOXYDATION D'UNE OLEFINE

(84) Designated Contracting States:
**AT BE BG CH CY CZ DE DK EE ES FI FR GB GR
IE IT LI LU MC NL PT SE SK TR**

(30) Priority: **20.11.2001 US 331874 P
20.11.2001 US 331828 P**

(43) Date of publication of application:
**22.09.2004 Bulletin 2004/39**

(73) Proprietor: **Shell Internationale Research
Maatschappij B.V.
2596 HR The Hague (NL)**

(72) Inventors:
• **BAKER, David, Scott
Houston, TX 77077 (US)**
• **KOBE, Jeffrey, Michael
Katy, TX 77450 (US)**
• **SCHUREN, John, George
Montgomery, TX 77356 (US)**
• **JOHNSON, Beamon, McNeil
Village Mills, TX 77663 (US)**
• **RUBINSTEIN, Leonid, Isaakovich
Houston, TX 77006 (US)**

(74) Representative: **Zeestraten, Albertus W. J.
Shell Internationale Research Maatschappij B.V.
P.O.Box 302
2501 CH The Hague (NL)**

(56) References cited:
**EP-A- 0 352 850       WO-A-97/10232
WO-A-97/28142       CN-A- 1 286 689**

• **SERAFIN, J.G., LIU, A.C., SEYEDMONIR, S.R.:
"Surface science and the silver-catalyzed
epoxidation of ethylene: an industrial
perspective" JOURNAL OF MOLECULAR
CATALYSIS A: CHEMICAL, vol. 131, 1998, pages
157-168, XP002230219**

## Description

Field of the Invention

The invention relates to a process for the epoxidation of an olefin, which process comprises reacting a feed comprising the olefin, oxygen and a reaction modifier in the presence of a highly selective silver-based catalyst. The invention also relates to systems suitable for use in connection with the process.

Background of the Invention

The catalytic epoxidation of olefins using a silver-based catalyst has been known for a long time. Conventional silver-based catalysts have provided the olefin oxides notoriously in a low selectivity. For example, when using conventional catalysts in the epoxidation of ethylene, the selectivity towards ethylene oxide, expressed as a fraction of the ethylene converted, does not reach values above the $^6/_7$ or 85.7 mole-% limit. Therefore, this limit has long been considered to be the theoretically maximal selectivity of this reaction, based on the stoichiometry of the reaction equation

$$7 \ C_2H_4 + 6 \ O_2 => 6 \ C_2H_4O + 2 \ CO_2 + 2 \ H_2O,$$

cf. Kirk-Othmer's *Encyclopedia of Chemical Technology,* 3$^{rd}$ ed., Vol. 9, 1980, p. 445.

Modern silver-based catalysts however are more selective towards olefin oxide production. When using the modern catalysts in the epoxidation of ethylene the selectivity towards ethylene oxide can reach values above the 6/7 or 85.7 mole-% limit referred to. Such highly selective catalysts, which may comprise as their active components silver, rhenium, at least one further element and optionally a rhenium co-promoter, are disclosed in EP-A-266015 and in several subsequent patent publications.

Besides better catalysts, reaction modifiers have been found which may be added to the feed to improve the selectivity (cf. for example EP-A-352850). Such reaction modifiers suppress the undesirable oxidation of olefin or olefin oxide to carbon dioxide and water, relative to the desired formation of olefin oxide, by a so-far unexplained mechanism. Suitable reaction modifiers are for example organic halides.

When applying a reaction modifier, the concentration of the reaction modifier in the feed may be chosen such that the selectivity is at the optimum value. The concentration at which the selectivity is at optimum may be found during the operation of the epoxidation process by a trial-and-error procedure, viz. by stepwise changing the reaction modifier supply rate and monitoring the effect on the selectivity. Such a procedure, however, would be cumbersome, and it would keep the process operating for some time at conditions which are less than the most economical. Moreover, the trial-and-error procedure would need to be redone when the feed composition has changed, in order to adjust the concentration of the reaction modifier to the new reaction conditions.

Summary of the Invention

The present invention enables the operator of an epoxidation process to avoid unwanted variations in the selectivity when the feed composition changes by changing the concentration of the reaction modifier such that a relative quantity Q of the reaction modifier is maintained substantially, preferably completely, at a constant level. Herein, the relative quantity Q is basically the ratio of the molar quantity of the reaction modifier in the feed to the molar quantity of hydrocarbons in the feed. In other words, the teaching of the present invention is that the concentration of the reaction modifier needed to achieve a certain effect on the selectivity is proportional to the concentration of the hydrocarbons present in the feed and, as a consequence, this effect on the selectivity may be preserved when changing the feed composition by also changing the reaction modifier concentration proportionally or substantially proportionally with any change in the hydrocarbon concentration. This is independent of any change in the feed composition other than changes relating to the hydrocarbons and/or the reaction modifier. Thus, it is a benefit of the present invention that it allows the epoxidation process to be controlled significantly more simply and more smoothly than without the invention.

It has also been found that in a quantitative sense there may be differences in the behavior of the various hydrocarbons which may be present in the reaction mixture, and it is therefore more preferred, when calculating Q, to replace the molar quantity of hydrocarbons by a -so-called-effective molar quantity of hydrocarbons. The effective molar quantity of hydrocarbons in the feed can be calculated from the feed composition, as set out hereinafter, such that it accounts for differences in the behavior between the various hydrocarbons present.

Further, it has been found that in a quantitative sense there may also be differences in the behavior of different reaction modifiers, while in practice a mixture of reaction modifiers is frequently present. Therefore it may be preferred, when calculating Q, also to replace the molar quantity of the reaction modifier by a -so-called- effective molar quantity of active species of the reaction modifier. The effective molar quantity of active species of the reaction modifier in the feed can be calculated from the feed composition, as set out hereinafter, such that it accounts for the differences in the behavior of different reaction modifiers.

Not wishing to be bound by theory, it is thought that, unlike other components of the feed, the hydrocarbons (for example, the olefin and saturated hydrocarbons, if present) have an ability to remove or strip reaction modifier from the catalyst. It is the teaching of this invention that, in order to maintain the effects of the reaction modifier, it is the concentration of the modifier's active species on the catalyst which needs to be maintained, as opposed to the concentration of the reaction modifier at places in the reaction mixture other than the catalyst surface. Differences in the ability of the various hydrocarbons in the removing/stripping process and differences in the effectiveness of the various reaction modifiers and their susceptibility to the removing/stripping process are thought to be accounted for by calculating and applying the effective molar quantities, as explained hereinbefore.

Accordingly, the present invention provides a process for the epoxidation of an olefin, which process comprises reacting a feed comprising the olefin, oxygen and a reaction modifier in the presence of a silver-based catalyst with the reaction modifier being present in a relative quantity Q which is the ratio of an effective molar quantity of active species of the reaction modifier present in the feed to an effective molar quantity of hydrocarbons present in the feed, and which process comprises the steps of:

- operating at a first operating phase wherein the value of Q is $Q_1$, and
- subsequently operating at a second operating phase wherein the feed composition is different from the feed composition employed in the first operating phase, such that the value of Q is $Q_2$, whereby the value of the quotient $Q_2/Q_1$ is in the range of from 0.5 to 1.5.

In a particular embodiment, the second phase is operated at a hydrocarbon composition and a reaction modifier composition of the feed of which at least one is different from the hydrocarbon composition and the reaction modifier composition of the feed employed in the first operating phase.

The invention also provides a system suitable for performing the process of this invention, which system comprises a reactor which holds a silver-based catalyst, means for feeding to the reactor a feed comprising the olefin, oxygen and a reaction modifier, and feed control means for controlling the feed and/or the feed composition, comprising modifier control means for controlling the reaction modifier being present in the feed in a relative quantity Q which is the ratio of an effective molar quantity of active species of the reaction modifier present in the feed to an effective molar quantity of hydrocarbons present in the feed, and which feed control means are configured such as to control the process steps of:

- operating at a first operating phase wherein the value of Q is $Q_1$, and
- subsequently operating at a second operating phase wherein the feed composition is different from the feed composition employed in the first operating phase, such that the value of Q is $Q_2$, whereby the value of the quotient $Q_2/Q_1$ is in the range of from 0.5 to 1.5.

The invention also provides a computer program product which comprises a computer readable medium and a computer readable program code, recorded on the computer readable medium, suitable for instructing a data processing system of a computer system to execute calculations for the process of this invention.

The invention also provides a computer system which comprises the computer program product of this invention and a data processing system configured to receive instructions read from the computer program product.

The invention also provides, in more general terms, a process for the epoxidation of an olefin, which process comprises reacting a feed comprising the olefin, oxygen and a reaction modifier in the presence of a silver-based catalyst, which process comprises the steps of:

- operating at a first operating phase, and
- subsequently operating at a second operating phase wherein the feed composition is different from the feed composition employed in the first operating phase, such that the concentration of active species of the reaction modifier on the catalyst is substantially unchanged.

Detailed Description of the Invention

Although the present epoxidation process may be carried out in many ways, it is preferred to carry it out as a gas phase process, i.e. a process in which the feed is contacted in the gas phase with the catalyst which is present as a solid material, typically in a packed bed. Generally the process is carried out as a continuous process. Frequently, in commercial scale operation, the process of the invention may involve a quantity of catalyst which is at least 10 kg, for example at least 20 kg, frequently in the range of from $10^2$ to $10^7$ kg, more frequently in the range of from $10^3$ to $10^6$ kg.

The olefin for use in the present epoxidation process may be any olefin, such as an aromatic olefin, for example styrene, or a di-olefin, whether conjugated or not, for example 1,9-decadiene or 1,3-butadiene. Typically, the olefin is a monoolefin, for example 2-butene or isobutene. Preferably, the olefin is a mono-α-olefin, for example 1-butene or propylene. The most preferred olefin is ethylene.

The olefin concentration in the feed is not material to this invention and may be selected within a wide range. Typically, the olefin concentration in the feed will be at most 80 mole-%, relative to the total feed. Preferably, it will be in the range of from 0.5 to 70 mole-%, in particular from 1 to 60 mole-%, on the same basis. As used herein, the feed is considered to be the composition which is contacted with the catalyst.

The present epoxidation process may be air-based or oxygen-based, see Kirk-Othmer's *Encyclopedia of Chemical Technology,* 3rd ed., Vol. 9, 1980, p. 445-447. In the air-based process air or air enriched with oxygen is employed as the source of the oxidizing agent while in the oxygen-based processes high-purity (>95 mole-%) oxygen is employed as the source of the oxidizing agent. Presently most epoxidation plants are oxygen-based and this is a preferred embodiment of the present invention.

The oxygen concentration in the feed is not material to this invention and may be selected within a wide range. However, in practice, oxygen is generally applied at a concentration which avoids the flammable regime. Typically, the concentration of oxygen applied will be within the range of from 1 to 15 mole-%, more typically from 2 to 12 mole-% of the total feed.

In order to remain outside the flammable regime, the concentration of oxygen in the feed may be lowered as the concentration of the olefin is increased. The actual safe operating ranges depend, along with the feed composition, also on the reaction conditions such as the reaction temperature and the pressure.

The reaction modifier is present in the feed for increasing the selectivity, suppressing the undesirable oxidation of olefin or olefin oxide to carbon dioxide and water, relative to the desired formation of olefin oxide. Many organic compounds, especially organic halides and organic nitrogen compounds, may be employed as the reaction modifier. Nitrogen oxides, hydrazine, hydroxylamine or ammonia may be employed as well. It is frequently considered that under the operating conditions of olefin epoxidation the nitrogen containing reaction modifiers are precursors of nitrates or nitrites, i.e. they are so-called nitrate- or nitrite-forming compounds (cf. e.g. EP-A-3642, US-A-4822900).

Organic halides are the preferred reaction modifiers, in particular organic bromides, and more in particular organic chlorides. Preferred organic halides are chlorohydrocarbons or bromohydrocarbons. More preferably they are selected from the group of methyl chloride, ethyl chloride, ethylene dichloride, ethylene dibromide, vinyl chloride or a mixture thereof. Most preferred reaction modifiers are ethyl chloride and ethylene dichloride.

Suitable nitrogen oxides are of the general formula $NO_x$ wherein x, which denotes the ratio of the number of oxygen atoms to the number of nitrogen atoms, is in the range of from 1 to 2. These nitrogen oxides include for example NO, $N_2O_3$ and $N_2O_4$. Suitable organic nitrogen compounds are nitro compounds, nitroso compounds, amines, nitrates and nitrites, for example nitromethane, 1-nitropropane or 2-nitropropane. In preferred embodiments, nitrate- or nitrite-forming compounds, e.g. nitrogen oxides and/or organic nitrogen compounds, are used together with an organic halide, in particular an organic chloride.

Although the reaction modifier may be supplied as a single compound, upon contact with the catalyst a variety of compounds may be formed which function as reaction modifier, and which may be present in the feed if a recycle is applied. For example, when applying ethyl chloride in an ethylene oxide process, the feed may in practice comprise ethyl chloride, vinyl chloride, ethylene dichloride and methyl chloride.

The reaction modifiers are generally effective when used in low concentration in the feed, for example up to 0.1 mole-%, relative to the total feed, for example from $0.01 \times 10^{-4}$ to 0.01 mole-%. In particular when the olefin is ethylene, it is preferred that the reaction modifier is present in the feed at a concentration of from $0.05 \times 10^{-4}$ to $50 \times 10^{-4}$ mole-%, in particular from $0.2 \times 10^{-4}$ to $30 \times 10^{-4}$ mole-%, relative to the total feed.

In addition to the olefin, oxygen and the reaction modifier, the feed may contain one or more optional components, such as carbon dioxide, water, inert gases and saturated hydrocarbons. Carbon dioxide and water are by-products of the epoxidation process. Carbon dioxide generally has an adverse effect on the catalyst activity. Typically, a concentration of carbon dioxide in the feed in excess of 25 mole-%, preferably in excess of 10 mole-%, relative to the total feed, is avoided. A concentration of carbon dioxide as low as 1 mole-% or lower, relative to the total feed, may be employed. Water may be introduced in the feed as a result of the recovery of olefin oxide and carbon dioxide from the reaction product. Water generally has an adverse effect on the catalyst activity. Typically, a concentration of water in the feed in excess of 3 mole-%, preferably in excess of 1 mole-%, relative to the total feed, is avoided. A concentration of water as low as 0.2 mole-% or lower, relative to the total feed, may be employed. Inert gas, for example nitrogen or argon, or a mixture thereof, may be present in the feed in a concentration of from 0.5 to 95 mole-%. In an air based process inert gas may be present in the feed in a concentration of from 30 to 90 mole-%, typically from 40 to 80 mole-%. In an oxygen based process inert gas may be present in the feed in a concentration of from 0.5 to 30 mole-%, typically from 1 to 15 mole-%. Suitable saturated hydrocarbons are propane and cyclopropane, and in particular methane and ethane. If saturated hydrocarbons are present, they may be present in a quantity of up to 80 mole-%, relative to the total feed, in particular up to 75 mole-%. Frequently they are present in a quantity of at least 30 mole-%, more frequently at least 40 mole-%. Saturated hydrocarbons may be added to the feed in order to increase the oxygen flammability limit.

The relative quantity Q of the reaction modifier is the ratio of the effective molar quantity of active species of the reaction modifier present in the feed to the effective molar quantity of hydrocarbons present in the feed, both molar quantities being expressed in the same units, for example as mole-%, based on the total feed.

When the reaction modifier is a halogen compound, for the purpose of calculating the effective molar quantity of active species of the reaction modifier and the value of Q, the number of active species is deemed to be the number of halogen atoms, and when the reaction modifier is a nitrate- or nitrite-forming compound, the number of active species is deemed to be the number of nitrogen atoms. This implies, for example, that 1 mole of ethylene dichloride provides 2 moles of active species, i.e. all of the chlorine atoms present provide an active species. On the other hand, it has also been found that reaction modifiers which are methyl compounds, such as methyl chloride and methyl bromide, are less responsive and therefore from 2 to 5 moles, in particular from 2.5 to 3.5 moles, suitably 3 moles of the methyl compounds may be deemed to provide 1 mole of the active species. This number may be determined and verified by routine experimentation, and -without wishing to be bound by theory- it is believed that this number is higher as the methyl compound in question has a lesser ability to split off the heteroatom in question (for example the halogen or nitrogen atom). Thus, for example, when the feed comprises $2\times10^{-4}$ mole-% of ethyl chloride, $3\times10^{-4}$ mole-% of vinyl chloride, $1\times10^{-4}$ mole-% of ethylene dichloride and $1.5\times10^{-4}$ mole-% of methyl chloride, the effective molar quantity of active species of the reaction modifier may be calculated to amount to $2\times10^{-4} \times 1 + 3\times10^{-4} \times 1 + 1\times10^{-4} \times 2 + 1.5\times10^{-4} \times \frac{1}{3} = 7.5\times10^{-4}$ mole-%.

Summarizing, the effective molar quantity of active species of the reaction modifier present in the feed may be calculated by multiplying the molar quantity of each of the reaction modifiers present in the feed with a factor, and adding up the resulting multiplication products, wherein each factor represents the number of active heteroatoms, in particular halogen atoms and/or nitrogen atoms, present per molecule of the reaction modifier in question, on the understanding that the factor for a reaction modifier which is a methyl compound may be in the range of from $\frac{1}{5}$ to ½, more typically from $\frac{1}{3.5}$ to $\frac{1}{2.5}$, suitably $\frac{1}{3}$.

The hydrocarbons present in the feed comprise the olefin and any saturated hydrocarbon present. As indicated herein-before, it is thought that the hydrocarbons present in the feed have the ability to remove/strip reaction modifier from the catalyst surface and the extent to which they have this ability may differ for the various hydrocarbons. In order to account for these differences (relative to ethylene), the molar quantity of each of the hydrocarbons present is multiplied with a factor, before the molar quantities are added up to calculate the effective molar quantity of the hydrocarbons. Herein, the factor of ethylene is 1, by definition; the factor for methane may be at most 0.5, or at most 0.4, typically in the range of from 0 to 0.2, more typically in the range of from 0 to 0.1; the factor for ethane may be in the range of from 50 to 150, more typically from 70 to 120; and the factor for higher hydrocarbons (i.e. having at least 3 carbon atoms) may be in the range of from 10 to 10000, more typically from 50 to 2000. Such factors may be determined and verified by routine experimentation, and -without wishing to be bound by theory- it is believed that the factor is higher as the hydrocarbon in question has a greater ability to form radicals. Suitable factors for methane, ethane, propane and cyclopropane, relative to ethylene, are 0.3, 85, 1000, and 60, respectively. As an example, when the feed comprises 30 mole-% ethylene, 40 mole-% of methane, 0.4 mole-% of ethane and 0.0001 mole-% of propane, the effective molar quantity of the hydrocarbons may be calculated to amount to $30 \times 1 + 40 \times 0.1 + 0.4 \times 85 + 0.0001 \times 1000 = 68.1$ mole-%.

It is noted that when ethylene oxide is produced from ethylene without further hydrocarbons being present, the effective molar quantity of the hydrocarbons equals the actual molar quantity, and that the addition of ethane or higher hydrocarbons to an ethylene feed contributes significantly to the effective molar quantity, whereas there is relatively little contribution from any methane added.

Eligible values of Q are at least $1\times10^{-6}$, and in particular at least $2\times10^{-6}$. Eligible values of Q are at most $100\times10^{-6}$, and in particular at most $50\times10^{-6}$.

In any operating phase of the process the concentration of the reaction modifier and, thus, the value of Q may be adjusted so as to achieve an optimal selectivity towards the olefin oxide formation, for the prevailing reaction conditions. This relates in particular to embodiments of this invention in which the catalyst is a highly selective silver based catalyst, as defined hereinafter.

In accordance with this invention, when the feed composition changes, the concentration of the reaction modifier may be changed as well, such that the value of Q is not substantially changed, which means that $Q_1$ is approximately equal, or preferably equal to $Q_2$. Preferably, the value of the quotient $Q_2/Q_1$ is in the range of from 0.8 to 1.2, in particular from 0.9 to 1.1, more in particular from 0.95 to 1.05. Most preferably the value of the quotient $Q_2/Q_1$ equals 1.

In this way the present invention enables to determine by calculation a desirable composition of the reaction modifier(s) or the hydrocarbon(s) in the feed which may be applied in the second operating phase of the epoxidation process, in response to a change in the feed composition as regards the quantity or type of the components of the feed. In a preferred embodiment, a desirable concentration of the reaction modifier(s) in the feed is calculated which may be applied in the second operating phase, in response to a change in the quantity or type of hydrocarbon(s) present in the feed. In another embodiment, a desirable concentration of the hydrocarbon(s) in the feed is calculated which may be applied in the second operating phase, in response to a change in the quantity or type of the reaction modifier(s) present in the feed. In again another embodiment, a desirable concentration of the reaction modifier(s) in the feed is calculated which may be applied in the second operating phase, in response to a change in the type of the reaction modifier(s) present in the feed, while it is preferred not to change the quantity or type of the hydrocarbon(s). In again another embodiment, a desirable concentration of the hydrocarbon(s) in the feed is calculated which may be applied in the second operating phase, in response to a change in the type of hydrocarbon(s) present in the feed, while it is preferred not to change the quantity or type of the reaction modifier(s).

There may be various reasons for the occurrence of changes in the feed composition. For example, by changing the oxygen concentration or the olefin concentration the production rate of the olefin oxide may be controlled, or changing the concentration of saturated hydrocarbons may change the flammability limits. The carbon dioxide concentration in the feed may change as a result of catalyst ageing. The concentration of inert gases in the feed may change as a result of a change in the concentration of such gases in the oxygen supplied to the process. Further, when a high selectivity catalyst is applied, the selectivity may be improved at an advanced stage of ageing of the catalyst by increasing the ethylene content of the feed (cf. US-6372925-B1 and WO-A-01/96324, i.e. non-prepublished PCT patent application PCT/US01/18097). By applying this invention when the process is operating at optimal selectivity, the change in the feed composition leads to less deviation of the selectivity from the optimum or even the selectivity may be maintained at the optimum level. By applying this invention when the process is not operating at optimal selectivity, application of this invention prevents the process from deviating further away from the optimum selectivity.

Any change in the feed composition may be gradual, or step wise, and any change in the feed composition may be accompanied with a corresponding change in the concentration of the reaction modifier, such that the value of Q is substantially unchanged or it is kept constant. Generally, the change in the feed composition is accompanied with a cocurrent change in the concentration of the reaction modifier.

The present epoxidation process may be carried out using reaction temperatures selected from a wide range. Preferably the reaction temperature is in the range of from 180 to 340 °C, more preferably in the range of from 190 to 325 °C, in particular in the range of from 200 to 300 °C. Preferably, as the catalyst ages, the reaction temperature is slowly increased as to compensate for a reduction in the activity of the catalyst. However, it is preferred that the change in the feed composition occurs substantially without a change in the reaction temperature. Frequently a change in reaction temperature, if any, accompanying the change in the feed composition, may be less than 10 °C, more frequently less than 5 °C, in particular less than 2 °C. Most preferably, the change in the feed composition is effected without a change in the reaction temperature at all.

Generally, the silver based catalyst is a supported catalyst. The support may be selected from a wide range of inert support materials. Such support materials may be natural or artificial inorganic materials and they include silicon carbide, clays, pumice, zeolites, charcoal and alkaline earth metal carbonates, such as calcium carbonate. Preferred are refractory support materials, such as alumina, magnesia, zirconia and silica. The most preferred support material is α-alumina.

The support material is preferably porous and has preferably a surface area, as measured by the B.E.T. method, of less than 20 $m^2$/g and in particular from 0.05 to 20 $m^2$/g. More preferably the B.E.T. surface area of the support is in the range of 0.1 to 10, in particular from 0.1 to 3.0 $m^2$/g. As used herein, the B.E.T. surface area is deemed to have been measured by the method as described in Brunauer, Emmet and Teller in *J. Am. Chem . Soc*. 60 (1938) 309-316.

The silver based catalyst preferably comprises silver and a further element or compound thereof. Eligible further elements are selected from the group of nitrogen, sulfur, phosphorus, boron, fluorine, Group IA metals, Group IIA metals, rhenium, molybdenum, tungsten, chromium, titanium, hafnium, zirconium, vanadium, thallium, thorium, tantalum, niobium, gallium and germanium and mixtures thereof. Preferably the Group IA metals are selected from lithium, potassium, rubidium and cesium. Most preferably the Group IA metal is lithium, potassium and/or cesium. Preferably the Group IIA metals are selected from calcium and barium. Where possible, the further element may suitably be provided as an oxyanion, for example as a sulfate, borate, perrhenate, molybdate or nitrate, in salt or acid form.

It is preferred to employ highly selective silver-based catalysts. The highly selective silver-based catalysts comprise, in addition to silver, one or more of rhenium, molybdenum, tungsten, a Group IA metal, and a nitrate- or nitrite-forming compound, which may each be present in a quantity of from 0.01 to 500 mmole/kg, calculated as the element (rhenium, molybdenum, tungsten, the Group IA metal or nitrogen) on the total catalyst. The nitrate- or nitrite-forming compounds and particular selections of nitrate- or nitrite-forming compounds are as defined hereinbefore. Rhenium, molybdenum, tungsten or the nitrate- or nitrite-forming compound may suitably be provided as an oxyanion, i.e. as a perrhenate, molybdate, tungstate or nitrate, in salt or acid form.

Of special preference are the highly selective silver based catalysts which comprise rhenium in addition to silver. Such catalysts are known from EP-A-266015. Broadly, they comprise silver, rhenium or compound thereof, the further element (as defined hereinbefore) other than rhenium or compound thereof and optionally a rhenium co-promoter which may be selected from one or more of sulfur, phosphorus, boron, and compounds thereof.

Preferred amounts of the components of the silver based catalysts are, when calculated as the element on the total catalyst:

- silver from 10 to 500 g/kg,
- if present, rhenium from 0.01 to 50 mmole/kg,
- if present, the further element or elements from 0.1 to 500 mmole/kg each, and,
- if present, the rhenium co-promoter or co-promoters from 0.1 to 30 mmole/kg each.

The present epoxidation process is preferably carried out at a reactor inlet pressure in the range of from 1000 to 4000 kPa. "GHSV" or Gas Hourly Space Velocity is the unit volume of gas at normal temperature and pressure (0 °C, 1 atm, i.e. 101.3 kPa) passing over one unit volume of packed catalyst per hour. Preferably, when the epoxidation process is as a gas phase process involving a packed catalyst bed, the GHSV is in the range of from 1500 to 10000 Nl/(l.h). Preferably, the process of this invention is carried out at a work rate in the range of from 0.5 to 10 kmole olefin oxide produced per $m^3$ of catalyst per hour, in particular 0.7 to 8 kmole olefin oxide produced per $m^3$ of catalyst per hour, for example 5 kmole olefin oxide produced per $m^3$ of catalyst per hour. As used herein, the work rate is the amount of the olefin oxide produced per unit volume of catalyst per hour and the selectivity is the molar quantity of the olefin oxide formed relative to the molar quantity of the olefin converted.

The olefin oxide produced may be recovered from the reaction product by using methods known in the art, for example by absorbing the olefin oxide from a reactor outlet stream in water and optionally recovering the olefin oxide from the aqueous solution by distillation. At least a portion of the aqueous solution containing the olefin oxide may be applied in a subsequent process for converting the olefin oxide into a 1,2-diol or a 1,2-diol ether.

The olefin oxide produced in the present epoxidation process may be converted into a 1,2-diol or into a 1,2-diol ether. As this invention leads to a more attractive process for the production of the olefin oxide, it concurrently leads to a more attractive process which comprises producing the olefin oxide in accordance with the invention and the subsequent use of the obtained olefin oxide in the manufacture of the 1,2-diol and/or 1,2-diol ether.

The conversion into the 1,2-diol or the 1,2-diol ether may comprise, for example, reacting the olefin oxide with water, suitably using an acidic or a basic catalyst. For example, for making predominantly the 1,2-diol and less 1,2-diol ether, the olefin oxide may be reacted with a ten fold molar excess of water, in a liquid phase reaction in presence of an acid catalyst, e.g. 0.5-1.0 %w sulfuric acid, based on the total reaction mixture, at 50-70 °C at 1 bar absolute, or in a gas phase reaction at 130-240 °C and 20-40 bar absolute, preferably in the absence of a catalyst. If the proportion of water is lowered the proportion of 1,2-diol ethers in the reaction mixture is increased. The 1,2-diol ethers thus produced may be a di-ether, tri-ether, tetra-ether or a subsequent ether. Alternative 1,2-diol ethers may be prepared by converting the olefin oxide with an alcohol, in particular a primary alcohol, such as methanol or ethanol, by replacing at least a portion of the water by the alcohol.

The 1,2-diol and the 1,2-diol ether may be used in a large variety of industrial applications, for example in the fields of food, beverages, tobacco, cosmetics, thermoplastic polymers, curable resin systems, detergents, heat transfer systems, etc.

Unless specified otherwise, the organic compounds mentioned herein, for example the olefins, 1,2-diols, 1,2-diol ethers and reaction modifiers, have typically at most 40 carbon atoms, more typically at most 20 carbon atoms, in particular at most 10 carbon atoms, more in particular at most 6 carbon atoms. As defined herein, ranges for numbers of carbon atoms (i.e. carbon number) include the numbers specified for the limits of the ranges.

The computer program product of this invention comprises a computer readable medium and a computer readable program code, recorded on the computer readable medium, suitable for instructing a data processing system to execute the calculations in connection with the process of this invention. In a preferred embodiment, the computer program product comprises, in addition, a computer readable program code, recorded on the computer readable medium, suitable for instructing the data processing system to control the process of this invention. The computer readable medium may be readable, for example, by means of an optical system or by means of a magnetic system. The computer program product may be in the form of a disk which is a permanent entity of the computer system of this invention, or it may be a disk which is insertable into the computer system. The feed control means may be configured such that they communicate with the computer system of this invention facilitating the control of the process steps of the process of this invention.

The following example will illustrate the invention.

Example (hypothetical)

A catalyst, as defined in EP-A-266015, containing silver, rhenium, cesium, lithium and sulfur on α-alumina and having a theoretical selectivity $S_0$ of 93% in the fresh state is employed in the following experiment. The above value of $S_0$ was determined by measuring selectivities in a range of gas hourly space velocities, each time at 30% ethylene, 8% oxygen, 5% carbon dioxide and 1400 kPa, the reaction temperature being 260 °C, and extrapolating back to zero oxygen conversion.

In the experiment ethylene oxide is produced as follows. A 1-kg sample of the catalyst is loaded into a tubular reactor consisting of a stainless steel tube. The tube is immersed in a boiling kerosene containing cooling jacket and the ends are connected to a gas flow system. The inlet gas flow rate is adjusted as to achieve a gas hourly space velocity of 6800 Nl /(l.h). The inlet pressure is 2100 kPa (absolute). The feed to the reactor comprises ethylene at a concentration of 28 mole-%, oxygen at a concentration of 8 mole-%, carbon dioxide at a concentration of 3 mole-%, ethane at a concentration of 0.5 mole-%, ethyl chloride at a concentration of $3\times10^{-4}$ mole-%, ethylene dichloride at a concentration of $0.5\times10^{-4}$ mole-%, vinyl chloride at a concentration of $1\times10^{-4}$ mole-%, and methyl chloride at a concentration of $2\times10^{-4}$ mole-%, and the remainder of the feed is nitrogen. The reaction temperature is 250 °C. The selectivity to ethylene oxide is optimal in respect of the chlorides. The value of Q (i.e. $Q_1$) equals $(3\times10^{-4} \times 1 + 0.5\times10^{-4} \times 2 + 1\times10^{-4} \times 1 + 2\times10^{-4} \times {}^1/_3) / (28 \times 1 + 0.5 \times 85) = 8.04\times10^{-6}$.

At a certain point in time the feed composition changes such that ethylene is present at a concentration of 25 mole-% and ethane at a concentration of 0.7 mole-%, the concentration of the other constituents being held constant, except ethyl chloride. In order to keep the selectivity at an optimum level, the ethyl chloride concentration is adjusted to $4.1\times10^{-4}$ mole-%. After these changes, the value of Q (i.e. $Q_2$) equals $(4.1\times10^{-4} \times 1 + 0.5\times10^{-4} \times 2 + 1\times10^{-4} \times 1 + 2\times10^{-4} \times {}^1/_3) / (25 \times 1 + 0.7 \times 85) = 8.01\times10^{-6}$.

**Claims**

1. A process for the epoxidation of an olefin, which process comprises reacting a feed comprising the olefin, oxygen and a reaction modifier in the presence of a silver-based catalyst with the reaction modifier being present in a relative quantity Q which is the ratio of an effective molar quantity of active species of the reaction modifier present in the feed to an effective molar quantity of hydrocarbons present in the feed, and which process comprises the steps of:

   - operating at a first operating phase wherein the value of Q is $Q_1$, and
   - subsequently operating at a second operating phase wherein the feed composition is different from the feed composition employed in the first operating phase, such that the value of Q is $Q_2$, whereby the value of the quotient $Q_2/Q_1$ is in the range of from 0.5 to 1.5.

2. A process as claimed in claim 1, wherein the olefin is ethylene, and wherein the reaction modifier comprises an organic chloride and optionally a nitrate- or nitrite-forming compound.

3. A process as claimed in claim 2, wherein the reaction modifier consists of chlorohydrocarbons having up to 10 carbon atoms, in particular up to 6 carbon atoms, which comprise one or more of methyl chloride, ethyl chloride, ethylene dichloride and vinyl chloride.

4. A process as claimed in any of claims 1-3, wherein the effective molar quantity of active species of the reaction modifier present in the feed can be calculated by multiplying the molar quantity of each of the reaction modifiers present in the feed with a factor, and adding up the resulting multiplication products, wherein each factor represents the number of active heteroatoms, in particular sulfur and/or nitrogen atoms, present per molecule of the reaction modifier in question, on the understanding that the factor for a reaction modifier which is a methyl compound, if present, is in the range of from ${}^1/_5$ to ${}^1/_2$, in particular in the range of from ${}^1/_{3.5}$ to ${}^1/_{2.5}$.

5. A process as claimed in any of claims 1-4, wherein the effective molar quantity of hydrocarbons present in the feed can be calculated by multiplying the molar quantity of each of the hydrocarbons present in the feed with a factor, and adding up the resulting multiplication products, wherein the factor for methane, if present, is in the range of from 0.1 to 0.5; the factor for ethane, if present, is in the range of from 50 to 150; and the factor for hydrocarbons having at least 3 carbon atoms, if present, is in the range of from 10 to 10000, all factors being relative to the factor for ethylene, which equals 1, by definition.

6. A process as claimed in claim 5, wherein the factor for methane, if present, is in the range of from 0 to 0.4; the factor for ethane, if present, is in the range of from 70 to 120; and the factor for the hydrocarbons having at least 3 carbon atoms, if present, is in the range of from 50 to 2000, all factors being relative to the factor for ethylene, which equals 1, by definition.

7. A process as claimed in any of claims 1-6, wherein the relative quantity Q is in the range of from $1\times10^{-6}$ to $100\times10^{-6}$, in particular in the range of from $2\times10^{-6}$ to $50\times10^{-6}$.

8. A process as claimed in any of claims 1-7, wherein the hydrocarbons present in the feed comprise one or more of methane, ethane, propane and cyclopropane, in addition to the olefin.

9. A process as claimed in any of claims 1-8, wherein such a value of $Q_1$ is employed that the selectivity towards the olefin oxide formation is optimal.

10. A process according to any of claims 1-9, wherein the value of the quotient $Q_2/Q_1$ is in the range of from 0.8 to 1.2, in particular in the range of from 0.9 to 1.1, more in particular in the range of from 0.95 to 1.05.

11. A process according to any of claims 1-10, wherein the second phase is operated at a hydrocarbon composition and a reaction modifier composition of the feed of which at least one is different from the hydrocarbon composition and the reaction modifier composition of the feed employed in the first operating phase.

12. A process according to claim 11, wherein the concentration of the reaction modifier(s) in the feed applied in the second operating phase is calculated, in response to a change in the quantity or type of hydrocarbon(s) present in the feed.

13. A process according to any of claims 1-12, wherein the silver based catalyst comprises silver and a further element or compound thereof, which further element is selected from the group of nitrogen, sulfur, phosphorus, boron, fluorine, Group IA metals, Group IIA metals, rhenium, molybdenum, tungsten, chromium, titanium, hafnium, zirconium, vanadium, thallium, thorium, tantalum, niobium, gallium and germanium and mixtures thereof, on a support, in particular an $\alpha$-alumina support.

14. A process according to claim 13, wherein the silver based catalyst comprises silver, rhenium or compound thereof, a further element or compound thereof which further element is selected from the group of nitrogen, sulfur, phosphorus, boron, fluorine, Group IA metals, Group IIA metals, molybdenum, tungsten, chromium, titanium, hafnium, zirconium, vanadium, thallium, thorium, tantalum, niobium, gallium and germanium and mixtures thereof, and optionally a rhenium co-promoter which may be selected from one or more of sulfur, phosphorus, boron, or compound thereof, on a support material, in particular an $\alpha$-alumina support.

15. A process according to claim 1 for the epoxidation of an olefin, which process comprises reacting a feed comprising the olefin, oxygen and a reaction modifier in the presence of a silver-based catalyst, which process comprises the steps of:

   - operating at a first operating phase, and
   - subsequently operating at a second operating phase wherein the feed composition is different from the feed composition employed in the first operating phase such that the concentration of active species of the reaction modifier on the catalyst is substantially unchanged.

16. A method for making a 1,2-diol or a 1,2-diol ether comprising converting an olefin oxide into the 1,2-diol or the 1,2-diol ether, wherein the olefin oxide has been obtained by a process for the production of olefin oxide according to any of claims 1-15.

17. A system suitable for performing a process according to any of claims 1-15, which system comprises a reactor which holds a silver-based catalyst, means for feeding to the reactor a feed comprising the olefin, oxygen and a reaction modifier, and feed control means for controlling the feed and/or the feed composition, comprising modifier control means for controlling the reaction modifier being present in the feed in a relative quantity Q which is the ratio of an effective molar quantity of active species of the reaction modifier present in the feed to an effective molar quantity of hydrocarbons present in the feed, and which feed control means are configured such as to control the process steps of:

   - operating at a first operating phase wherein the value of Q is $Q_1$, and
   - subsequently operating at a second operating phase wherein the feed composition is different from the feed composition employed in the first operating phase, such that the value of Q is $Q_2$, whereby the value of the quotient $Q_2/Q_1$ is in the range of from 0.5 to 1.5.

18. A computer program product which comprises a computer readable medium and a computer readable program code, recorded on the computer readable medium, suitable for instructing a data processing system of a computer system to execute calculations for the process according to any of claims 1-15.

19. A computer program product as claimed in claim 20, which comprises, in addition, a computer readable program code, recorded on the computer readable medium, suitable for instructing the data processing system to control a process according to any of claims 1-15.

20. A computer system which comprises a computer program product and a data processing system configured to receive instructions read from the computer program product, wherein the computer program product is as claimed in claim 18 or 19.

**Patentansprüche**

1. Verfahren zur Epoxidierung eines Olefins, welches Verfahren das Umsetzen eines Einsatzmaterials, das das Olefin, Sauerstoff und ein Reaktionsmodifikationsmittel umfaßt, in Gegenwart eines auf Silber basierenden Katalysators umfaßt, wobei das Reaktionsmodifikationsmittel in einer relativen Menge Q vorhanden ist, welche das Verhältnis einer effektiven molaren Menge an aktiver Spezies des im Einsatzmaterial vorhandenen Reaktionsmodifikationsmittels zu einer effektiven molaren Menge an im Einsatzmaterial vorhandenen Kohlenwasserstoffen ist, und welches Verfahren die Schritte von:

   - Durchführen einer ersten Betriebsphase, worin der Wert von Q $Q_1$ ist, und
   - darauffolgendes Durchführen einer zweiten Betriebsphase, worin die Einsatzmaterialzusammensetzung von der in der ersten Betriebsphase angewandten Einsatzmaterialzusammensetzung verschieden ist, sodaß der Wert von Q $Q_2$ ist, wodurch der Wert des Quotienten $Q_2/Q_1$ im Bereich von 0,5 bis 1,5 beträgt.

2. Verfahren nach Anspruch 1, worin das Olefin Ethylen ist und worin das Reaktionsmodifikationsmittel ein organisches Chlorid und wahlweise eine nitrat- oder nitritausbildende Verbindung umfaßt.

3. Verfahren nach Anspruch 2, worin das Reaktionsmodifikationsmittel aus Chlorkohlenwasserstoffen mit bis zu 10 Kohlenstoffatomen, insbesondere bis zu 6 Kohlenstoffatomen besteht, welche einen oder mehrere von Methylchlorid, Ethylchlorid, Ethylenchlorid und Vinylchlorid umfassen.

4. Verfahren nach einem der Ansprüche 1 bis 3, worin die effektive molare Menge an aktiven Spezies des im Einsatzmaterial vorhandenen Reaktionsmodifikationsmittels durch Multiplizieren der molaren Menge von jedem der im Einsatzmaterial vorhandenen Reaktionsmodifikationsmittel mit einem Faktor, und Addieren der resultierenden Multiplikationsergebnisse berechnet werden kann, wobei jeder Faktor die Anzahl an aktiven Heteroatomen darstellt, insbesondere an den jeweiligen Schwefel- und/oder Stickstoffatomen, welche pro Molekül des in Frage kommenden Reaktionsmodifikationsmittels vorhanden sind, unter der Voraussetzung, daß der Faktor für ein Reaktionsmodifikationsmittel, welches eine Methylverbindung ist, wenn diese vorhanden ist, im Bereich von 1/5 bis 1/2, insbesondere im Bereich von 1/3,5 bis 1/2,5 liegt.

5. Verfahren nach einem der Ansprüche 1 bis 4, worin die effektive molare Menge an im Einsatzmaterial vorhandenen Kohlenwasserstoffen durch Multiplizieren der molaren Menge von jedem der im Einsatzmaterial vorhandenen Kohlenwasserstoffe mit einem Faktor, und Addieren der erhaltenen Multiplikationsprodukte berechnet werden kann, wobei der Faktor für Methan, wenn dieses vorhanden ist, im Bereich von 0,1 bis 0,5 liegt; der Faktor für Ethan, wenn dieses vorhanden ist, im Bereich von 50 bis 150 liegt, und der Faktor für Kohlenwasserstoffe mit wenigstens 3 Kohlenstoffatomen, wenn diese vorhanden sind, im Bereich von 10 bis 10.000 liegt, wobei alle Faktoren relativ zum Faktor für Ethylen sind, welcher per Definition 1 entspricht.

6. Verfahren nach Anspruch 5, worin der Faktor für Methan, wenn dieses vorhanden ist, im Bereich von 0 bis 0,4 liegt, der Faktor für Ethan, wenn dieses vorhanden ist, im Bereich von 70 bis 120 liegt, und der Faktor für die Kohlenwasserstoffe mit wenigstens 3 Kohlenstoffatomen, wenn diese vorhanden sind, im Bereich von 50 bis 2.000 liegt, wobei alle Faktoren relativ zum Faktor für Ethylen sind, welcher per Definition 1 entspricht.

7. Verfahren nach einem der Ansprüche 1 bis 6, worin die relative Menge Q im Bereich von $1 \times 10^{-6}$ bis $100 \times 10^{-6}$, insbesondere im Bereich von $2 \times 10^{-6}$ bis $50 \times 10^{-6}$ liegt.

8. Verfahren nach einem der Ansprüche 1 bis 7, worin die im Einsatzmaterial vorhandenen Kohlenwasserstoffe einen oder mehrere von Methan, Ethan, Propan und Cyclopropan, zusätzlich zum Olefin umfassen.

9. Verfahren nach einem der Ansprüche 1 bis 8, worin ein derartiger Wert von $Q_1$ angewandt wird, daß die Selektivität hin zur Olefinoxidbildung optimal ist.

10. 'Verfahren nach einem der Ansprüche 1 bis 9, worin der Wert des Quotienten $Q_2/Q_1$ im Bereich von 0,8 bis 1,2, insbesondere im Bereich von 0,9 bis 1,1, in noch besonderer Weise im Bereich von 0,95 bis 1,05 liegt.

11. Verfahren nach einem der Ansprüche 1 bis 10, worin die zweite Phase bei einer Kohlenwasserstoffzusammensetzung und einer Reaktionsmodifikationsmittelzusammensetzung des Einsatzmaterials durchgeführt wird, wovon wenigstens eine von der Kohlenwasserstoffzusammensetzung und der Reaktionsmodifikationsmittelzusammensetzung des in der ersten Betriebsphase angewandten Einsatzmaterials verschieden ist.

12. Verfahren nach Anspruch 11, worin die Konzentration des Reaktionsmodifikationsmittels (der Reaktionsmodifikationsmittel) im Einsatzmaterial, welches in der zweiten Betriebsphase angewandt wird, als Antwort auf eine Änderung in der Menge oder dem Typ des (der) im Einsatzmaterial vorhandenen Kohlenwasserstoffs (Kohlenwasserstoffe), berechnet wird.

13. Verfahren nach einem der Ansprüche 1 bis 12, worin der auf Silber basierende Katalysator Silber und ein weiteres Element oder eine Verbindung hievon, welches weitere Element von der aus Stickstoff, Schwefel, Phosphor, Bor, Fluor, Gruppe IA-Metallen, Gruppe IIA-Metallen, Rhenium, Molybdän, Wolfram, Chrom, Titan, Hafnium, Zirconium, Vanadium, Thallium, Thorium, Tantal, Niob, Gallium und Germanium und Gemischen hievon bestehenden Gruppe ausgewählt ist, auf einem Träger, insbesondere einem α-Aluminiumoxidträger, umfaßt.

14. Verfahren nach Anspruch 13, worin der auf Silber basierende Katalysator Silber, Rhenium oder eine Verbindung hievon, ein weiteres Element oder eine Verbindung hievon, welches weitere Element von der aus Stickstoff, Schwefel, Phosphor, Bor, Fluor, Gruppe IA-Metallen, Gruppe IIA-Metallen, Molybdän, Wolfram, Chrom, Titan, Hafnium, Zirconium, Vanadium, Thallium, Thorium, Tantal, Niob, Gallium und Germanium und Gemischen hievon bestehenden Gruppe ausgewählt ist, und wahlweise einen Rhenium-Co-Promotor, welcher unter einem oder mehreren von Schwefel, Phosphor, Bor oder einer Verbindung hievon ausgewählt sein kann, auf einem Trägermaterial, insbesondere einem α-Aluminiumoxidträger, umfaßt.

15. Verfahren nach Anspruch 1 zur Epoxidierung eines Olefins, welches Verfahren das Umsetzen eines das Olefin, Sauerstoff und ein Reaktionsmodifikationsmittel umfassenden Einsatzmaterials in Gegenwart eines auf Silber basierenden Katalysators umfaßt, welches Verfahren die Schritte von:

- Durchführen einer ersten Betriebsphase, und
- darauffolgendes Durchführen einer zweiten Betriebsphase umfaßt, worin die Einsatzmaterialzusammensetzung von der in der ersten Betriebsphase angewandten Einsatzmaterialzusammensetzung verschieden ist, derart, daß die Konzentration an aktiven Spezies des Reaktionsmodifikationsmittels auf dem Katalysator im wesentlichen unverändert ist.

16. Verfahren zur Herstellung eines 1,2-Diols oder eines 1;2-Diolethers, umfassend das Umwandeln eines Olefinoxids in den 1,2-Diol oder den 1,2-Diolether, wobei das Olefinoxid durch ein Verfahren zur Herstellung eines Olefinoxids nach einem der Ansprüche 1 bis 15 erhalten wurde.

17. Ein System, welches zur Durchführung eines Verfahrens nach einem der Ansprüche 1 bis 15 geeignet ist, welches System einen Reaktor, der einen auf Silber basierenden Katalysator enthält, Mittel zum Zuführen eines Einsatzmaterials, welches das Olefin, Sauerstoff und ein Reaktionsmodifikationsmittel umfaßt, zu dem Reaktor, und Einsatzmaterialsteuerungsmittel zur Steuerung des Einsatzmaterials und/oder der Einsatzmaterialzusammensetzung umfaßt, umfassend Modifikationsmittelsteuerungsmittel zum Steuern des im Einsatzmaterial in einer relativen Menge Q vorhandenen Reaktionsmodifikationsmittels, welche relative Menge Q das Verhältnis einer effektiven molaren Menge an aktiven Spezies des im Einsatzmaterial vorhandenen Reaktionsmodifikationsmittels zu einer effektiven molaren Menge an im Einsatzmaterial vorhandenen Kohlenwasserstoffen ist, und welche Einsatzmaterialsteuerungsmittel derart konfiguriert sind, daß die Verfahrensschritte von:

- Durchführen einer ersten Betriebsphase, worin der Wert von Q $Q_1$ ist, und
- darauffolgendes Durchführen einer zweiten Betriebsphase, worin die Einsatzmaterialzusammensetzung von der in der ersten Betriebsphase angewandten Einsatzmaterialzusammensetzung verschieden ist, sodaß der Wert von Q $Q_2$ ist, wodurch der Wert des Quotienten $Q_2/Q_1$ im Bereich von 0,5 bis 1,5 liegt,

gesteuert werden.

18. Computerprogrammprodukt, welches ein computerlesbares Medium und einen computerlesbaren Programmcode umfaßt, welcher auf dem computerlesbaren Medium aufgezeichnet ist, welches zur Steuerung eines Datenverarbeitungssystems eines Computersystems geeignet ist, um Berechnungen für das Verfahren nach einem der Ansprüche 1 bis 15 auszuführen.

19. Computerprogrammprodukt nach Anspruch 20, welches zusätzlich einen computerlesbaren Programmcode, der auf einem computerlesbaren Medium aufgezeichnet ist, umfaßt, der zur Steuerung des Datenverarbeitungssystems zur Überwachung/Steuerung eines Verfahrens nach einem der Ansprüche 1 bis 15 geeignet ist.

20. Computersystem, welches ein Computerprogrammprodukt und ein Datenverarbeitungssystem umfaßt, welches konfiguriert ist, um vom Computerprogrammprodukt abgelesene Instruktionen zu erhalten, wobei das Computerprogrammprodukt ein solches ist, wie es in Anspruch 18 oder 19 beansprucht ist.

## Revendications

1. Procédé d'époxydation d'une oléfine, lequel procédé comprend la mise en réaction d'une charge comprenant l'oléfine, de l'oxygène et un modificateur de réaction en présence d'un catalyseur à base d'argent, le modificateur de réaction étant présent en quantité relative Q qui est le rapport d'une quantité molaire efficace d'espèces actives du modificateur de réaction présentes dans la charge à une quantité molaire efficace d'hydrocarbures présents dans la charge, lequel procédé comprend les étapes suivantes :

   - un fonctionnement dans une première phase opératoire dans laquelle la valeur de Q est $Q_1$, et
   - ensuite, un fonctionnement dans une seconde phase opératoire dans laquelle la composition de la charge est différente de la composition de la charge employée dans la première phase opératoire de sorte que la valeur de Q soit $Q_2$, la valeur du quotient $Q_2/Q_1$ se situant dans la plage de 0,5 à 1,5.

2. Procédé selon la revendication 1, dans lequel l'oléfine est l'éthylène et dans lequel le modificateur de réaction comprend un chlorure organique et éventuellement un composé formateur de nitrate ou de nitrite.

3. Procédé selon la revendication 2, dans lequel le modificateur de réaction est constitué de chlorohydrocarbures ayant jusqu'à 10 atomes de carbone, en particulier jusqu'à 6 atomes de carbone, qui comprennent un ou plusieurs des corps parmi le chlorure de méthyle, le chlorure d'éthyle, le dichlorure d'éthylène et le chlorure de vinyle.

4. Procédé selon l'une quelconque des revendications 1 à 3, dans lequel la quantité molaire efficace d'espèces actives du modificateur de réaction présentes dans la charge peut être calculée en multipliant la quantité molaire de chacun des modificateurs de réaction présents dans la charge par un facteur, et en ajoutant les produits de multiplication obtenus, où chaque facteur représente le nombre d'hétéroatomes actifs, en particulier d'atomes de soufre et/ou d'azote, présents par molécule du modificateur de réaction en question, à condition que le facteur pour un modificateur de réaction qui est un composé de méthyle, s'il y en a, se situe dans la plage de 1/5 à 1/2, en particulier dans la plage de 1/3,5 à 1/2,5.

5. Procédé selon l'une quelconque des revendications 1 à 4, dans lequel la quantité molaire efficace d'hydrocarbures présents dans la charge peut être calculée en multipliant la quantité molaire de chacun des hydrocarbures présents dans la charge par un facteur, et en ajoutant les produits de multiplication obtenus, où le facteur pour le méthane, s'il y en a, se situe dans la plage de 0,1 à 0,5 ; le facteur pour l'éthane, s'il y en a, se situe dans la plage de 50 à 150 ; et le facteur pour les hydrocarbures ayant au moins 3 atomes de carbone, s'il y en a, se situe dans la plage de10 à 10000, tous les facteurs se rapportant au facteur pour l'éthylène qui est égal à 1, par définition.

6. Procédé selon la revendication 5, dans lequel le facteur pour le méthane, s'il y en a, se situe dans la plage de 0 à 0,4 ; le facteur pour l'éthane, s'il y en a, se situe dans la plage de 70 à 120 ; et le facteur pour les hydrocarbures ayant au moins 3 atomes de carbone, s'il y en a, se situe dans la plage de 50 à 2000, tous les facteurs se rapportant au facteur pour l'éthylène, qui est égal à 1, par définition.

7. Procédé selon l'une quelconque des revendications 1 à 6, dans lequel la quantité relative Q se situe dans la plage de $1 \times 10^{-6}$ à $100 \times 10^{-6}$, en particulier dans la plage de $2 \times 10^{-6}$ à $50 \times 10^{-6}$.

8. Procédé selon l'une quelconque des revendications 1 à 7, dans lequel les hydrocarbures présents dans la charge comprennent un ou plusieurs des corps parmi le méthane, l'éthane, le propane et le cyclopropane, en plus de l'oléfine.

9. Procédé selon l'une quelconque des revendications 1 à 8, dans lequel cette valeur de $Q_1$ est employée de sorte que la sélectivité envers la formation d'oxyde d'oléfine soit optimale.

10. Procédé selon l'une quelconque des revendications 1 à 9, dans lequel la valeur du quotient $Q_2/Q_1$ se situe dans la plage de 0,8 à 1,2, en particulier dans la plage de 0,9 à 1,1, mieux encore dans la plage de 0,95 to 1,05.

11. Procédé selon l'une quelconque des revendications 1à 10, dans lequel la seconde phase est mise en oeuvre avec une composition d'hydrocarbure et une composition de modificateur de réaction de la charge dont au moins l'une est différente de la composition d'hydrocarbure et de la composition de modificateur de réaction de la charge employées dans la première phase opératoire.

12. Procédé selon la revendication 11, dans lequel la concentration du ou des modificateurs de réaction dans la charge appliquée dans la seconde phase opératoire est calculée en réponse à une variation de la quantité ou du type d'hydrocarbure(s) présent(s) dans la charge.

**13.** Procédé selon l'une quelconque des revendications 1 à 12, dans lequel le catalyseur à base d'argent comprend de l'argent et un autre élément ou un de ses composés, lequel autre élément est choisi dans le groupe constitué de l'azote, du soufre, du phosphore, du bore, du fluor, des métaux du groupe IA, des métaux du groupe IIA, du rhénium, du molybdène, du tungstène, du chrome, du titane, du hafnium, du zirconium, du vanadium, du thallium, du thorium, du tantale, du niobium, du gallium, du germanium et de leurs mélanges, sur un support, en particulier un support de α-alumine.

**14.** Procédé selon la revendication 13, dans lequel le catalyseur à base d'argent comprend de l'argent, du rhénium ou un de ses composés, un autre élément ou un de ses composés, lequel autre élément est choisi dans le groupe constitué de l'azote, du soufre, du phosphore, du bore, du fluor, des métaux du groupe IA, des métaux du groupe IIA, du molybdène, du tungstène, du chrome, du titane, du hafnium, du zirconium, du vanadium, du thallium, du thorium, du tantale, du niobium, du gallium, du germanium et de leurs mélanges, et éventuellement un co-promoteur de rhénium qui peut être choisi parmi un ou plusieurs des corps parmi le soufre, le phosphore, le bore, ou un de leurs composés, sur un matériau de support, en particulier un support de α-alumine.

**15.** Procédé selon la revendication 1 pour l'époxydation d'une oléfine, lequel procédé comprend la mise en réaction d'une charge comprenant l'oléfine, de l'oxygène et un modificateur de réaction en présence d'un catalyseur à base d'argent, lequel procédé comprend les étapes suivantes :

- un fonctionnement dans une première phase opératoire, et
- ensuite, un fonctionnement dans une seconde phase opératoire, dans laquelle la composition de la charge est différente de la composition de la charge employée dans la première phase opératoire de sorte que la concentration des espèces actives du modificateur de réaction sur le catalyseur soit sensiblement inchangée.

**16.** Procédé de production d'un 1,2-diol ou d'un éther de 1,2-diol, comprenant la conversion d'un oxyde d'oléfine en 1,2-diol ou en éther de 1,2-diol, dans lequel l'oxyde d'oléfine a été obtenu par un procédé de production d'un oxyde d'oléfine selon l'une quelconque des revendications 1 à 15.

**17.** Système convenant à la réalisation d'un procédé selon l'une quelconque des revendications 1 à 15, lequel système comprend un réacteur qui contient un catalyseur à base d'argent, des moyens pour introduire dans le réacteur une charge comprenant l'oléfine, de l'oxygène et un modificateur de réaction, et des moyens de réglage de charge pour régler la charge et/ou la composition de la charge, comprenant des moyens de réglage du modificateur pour régler le modificateur de réaction présent dans la charge en quantité relative Q, qui est le rapport d'une quantité molaire efficace d'espèces actives du modificateur de réaction présentes dans la charge, lesdits moyens de réglage de charge étant configurés de manière à régler les étapes suivantes du procédé :

- un fonctionnement dans une première phase opératoire dans laquelle la valeur de Q est $Q_1$, et
- ensuite, un fonctionnement dans une seconde phase opératoire, dans laquelle la composition de la charge est différente de la composition de la charge employée dans la première phase opératoire de sorte que la valeur de Q soit Q2, la valeur du quotient $Q_2/Q_1$ se situant dans la plage de 0,5 à 1,5.

**18.** Produit de programme informatique qui comprend un support lisible par ordinateur et un code de programme lisible par ordinateur enregistré sur le support lisible par ordinateur, convenant pour instruire un système de traitement de données d'un système informatique afin d'effectuer des calculs pour le procédé selon l'une quelconque des revendications 1 à 15.

**19.** Produit de programme informatique selon la revendication 20, qui comprend, de plus, un code de programme lisible par ordinateur, enregistré sur le support lisible par ordinateur convenant pour instruire le système de traitement de données afin de régler un procédé selon l'une quelconque des revendications 1 à 15.

**20.** Système informatique qui comprend un produit de programme informatique et un système de traitement de données configuré pour recevoir des instructions lues dans le produit de programme informatique, où le produit de programme informatique est tel que revendiqué dans la revendication 18 ou 19.